# EUROPEAN PATENT SPECIFICATION

(11) **EP 3 951 360 B1**
(45) Date of publication and mention of the grant of the patent: **27.12.2023**
(21) Application number: 20776310.3
(22) Date of filing: 12.03.2020
(51) Int. Cl.: G01N 21/47, G01N 21/59, G01N 33/543, G01N 35/00, G16B 25/10, G01N 21/55, G01N 21/75

(54) **ANALYSIS DEVICE AND ANALYSIS METHOD**
ANALYSEVORRICHTUNG UND ANALYSEVERFAHREN
DISPOSITIF D'ANALYSE ET PROCÉDÉ D'ANALYSE

(30) Priority: 25.03.2019 JP 2019056241
(43) Date of publication of application: 09.02.2022
(73) Proprietor: JVCKenwood Corporation, Yokohama-shi, Kanagawa 2210022 (JP)
(72) Inventor: SAITO, Atsushi, Yokohama-shi, Kanagawa 221-0022 (JP)
(74) Representative: Klang, Alexander H.
(86) International application number: PCT/JP2020/010703
(87) International publication number: WO 2020/195869

(56) References cited:
- WO-A1-2012/014778
- WO-A1-2014/200767
- WO-A1-2014/210223
- WO-A1-2017/056526
- WO-A1-2019/031514
- JP-A- 2005 522 698
- JP-A- 2006 292 410
- JP-A- 2008 529 024
- JP-A- 2009 080 096
- JP-A- 2014 119 418
- JP-A- 2014 119 418
- JP-A- 2014 149 216
- JP-A- 2017 058 242
- JP-A- 2017 058 242
- JP-A- 2017 138 186
- JP-A- 2017 207 289
- US-A1- 2015 247 797

## Description

### [TECHNICAL FIELD]

The present disclosure relates to an analysis device and an analysis method for analyzing biological materials such as antigens or antibodies.

### [BACKGROUND ART]

Immunoassays are known which are for quantitatively analyzing discovery of diseases, effects of medical treatment, and the like by detecting particular antigens or antibodies associated with the disease as biomarkers.

JP 2017-58242 A discloses a quantitative method for indirectly quantifying a detection target substance. Specifically, an analysis unit having a plurality of wells is used to fix an antibody on an analysis substrate, and a detection target substance is bound with the antibody. A reaction region on which the detection target substance is captured in a sandwiched manner is formed by binding fine particles for labeling the detection target substance with the detection target substance, and the number of the fine particles in the reaction region are counted. This enables the indirect quantitative determination of the detection target substance.

JP 2014 119418 A discloses that a correction coefficient indicating an apparatus individual difference corresponding to the type of a tested substance is stored in the storage means of a fluorescence detector in advance. Identification information about the type of the tested substance is obtained from an analysis chip. With a test body supplied to a first sensor area and a second sensor area, the state of connection or competition in the first sensor area is detected as a first signal, and the state of connection or competition in the second sensor area is detected as a second signal. In data analysis means, a third signal is calculated by normalizing the first signal by use of the second signal. The correction coefficient derived from the storage means according to identification information is calculated together with a third signal, and the device individual difference is corrected.

WO 2014 200767 A discloses methods, compositions, kits and systems for multiplexed detection of target molecules from a sample. In some embodiments, the methods, compositions, kits and systems can be used to perform multiplexed protein analysis of a sample (e.g., a sample comprising a small number of cells or a single-cell sample).

WO 2014 210223 A discloses methods and assay systems for use in spatially encoded biological assays, including assays to determine a spatial pattern of abundance, expression, and/or activity of one or more biological targets across multiple sites in a sample. In particular, the present disclosure provides methods and assay systems capable of high levels of multiplexing where reagents are provided to a biological sample in order to address tag the sites to which reagents are delivered; instrumentation capable of controlled delivery of reagents, in particular, microfluidic device based instrumentation; and a decoding scheme providing a readout that is digital in nature.

### [SUMMARY OF THE INVENTION]

If an analysis by an immunoassay is made by using the above described quantitative method, a plurality of antibodies are individually fixed to different reaction regions, the number of fine particles in each of the reaction region fixed with the individual antibodies is counted, and the ratio of determined count values (relative count value) is obtained. Accordingly, it is determined to which antibody the detection target substance is bound. However, the above analysis method is based on the assumption that the antibodies are uniformly fixed in the reaction regions on the analysis substrate. Therefore, if a situation occurs in which the antibodies are non-uniformly fixed, the accuracy of the relative count value may be deteriorated, and the accuracy of the analysis may be deteriorated.

An object of an embodiment of the present invention is to provide an analysis device and an analysis method capable of accurately performing the analysis by an immunoassay even if antibodies are non-uniformly fixed in individual reaction regions.

In accordance with the present invention, an analysis device as set forth in the appended claims is provided. In particular, a first aspect of an embodiment of the present invention provides an analysis device as defined in claim 1.

A second aspect of an embodiment of the present invention provides an analysis method as defined in claim 4.

According to an analysis device and an analysis method of an embodiment, in the analysis method using a relative count value, a detection target substance can be accurately analyzed even if how detection target substances are captured is different for each of reaction regions formed under the same condition.

### [BRIEF DESCRIPTION OF THE DRAWINGS]

[FIG. 1] FIG. 1 is a configuration diagram showing an example of an analysis device of an embodiment.
[FIG. 2] FIG. 2 is a diagram showing an example of a positional relationship between a detection target substance capturing region and a blank region in a reaction region.
[FIG. 3] FIG. 3 is a diagram schematically showing a state in which a detection target substance is captured on an analysis substrate by an antibody and fine particles in a detection target substance capturing region in a sandwiched manner.
[FIG. 4] FIG. 4 is a flowchart showing an example of an analysis method of an embodiment.
[FIG. 5] FIG. 5 is a diagram showing an example of the relationship among a reference position detection signal, a measurement gate signal GS, a light reception level signal, and a fine particle pulse signal.
[FIG. 6] FIG. 6 is a diagram showing a plurality of detection target substance capturing regions in a reaction region.
[FIG. 7] FIG. 7 is a diagram showing a plurality of blank regions in a reaction region.
[FIG. 8] FIG. 8 is a diagram showing a comparative example of in-plane distribution of count values in a detection target substance capturing region in a reaction region.
[FIG. 9] FIG. 9 is a diagram showing a comparative example of in-plane distribution of count values in a blank region in a reaction region.
[FIG. 10] FIG. 10 is a diagram showing a comparative example of in-plane distribution of count values in a detection target substance capturing region in a reaction region.
[FIG. 11] FIG. 11 is a diagram showing a comparative example of in-plane distribution of count values in a blank region in a reaction region.
[FIG. 12] FIG. 12 is a diagram showing an example of in-plane distribution of count values in a detection target substance capturing region and a blank region in a reaction region.
[FIG. 13] FIG. 13 is a diagram showing an example of in-plane distribution of count values in a detection target substance capturing region and a blank region in a reaction region.
[FIG. 14] FIG. 14 is a diagram showing an example of a positional relationship between a detection target substance capturing region and a blank region in a reaction region.
[FIG. 15] FIG. 15 is a diagram showing an example of a positional relationship between a detection target substance capturing region and a blank region in a reaction region.

### [MODES FOR CARRYING OUT THE INVENTION]

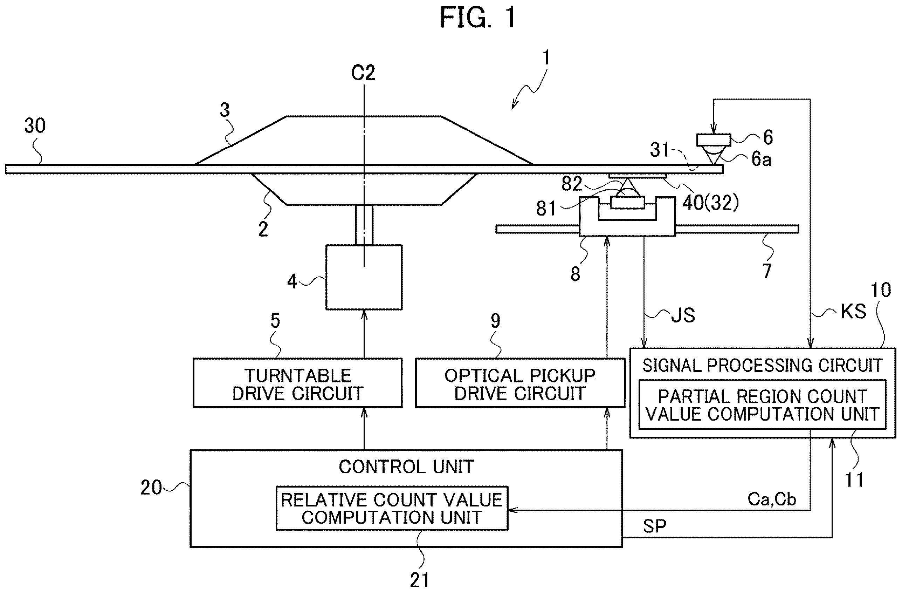

With reference to FIG. 1, a configuration example of an analysis device according to an embodiment will be described. An analysis device 1 includes a turntable 2, a clamper 3, a turntable drive unit 4, a turntable drive circuit 5, a reference position detection sensor 6, a guide shaft 7, an optical pickup 8, an optical pickup drive circuit 9, a signal processing circuit 10, and a control unit 20.

The analysis substrate 30 is placed on the turntable 2 such that a reaction region 40 formed on an analysis substrate 30 faces downward. The analysis substrate 30 is, for example, an optical disk having a disk shape. The clamper 3 is driven in a direction away from and close to the turntable 2. The analysis substrate 30 is held by the clamper 3 and the turntable 2.

The turntable drive unit 4 rotationally drives the turntable 2 around a rotation axis C2 together with the analysis substrate 30 and the clamper 3. A spindle motor may be used as the turntable drive unit 4. The turntable drive circuit 5 controls the turntable drive unit 4. For example, the turntable drive circuit 5 controls the turntable drive unit 4 such that the turntable 2 rotates at a constant linear velocity together with the analysis substrate 30 and the clamper 3.

The reference position detection sensor 6 is disposed in the vicinity of an outer peripheral portion of the analysis substrate 30. The reference position detection sensor 6 is, for example, an optical sensor such as a photoreflector. The reference position detection sensor 6 irradiates, with detecting light 6a, the outer peripheral portion of the rotating analysis substrate 30. Then, the reference position detection sensor 6 receives reflected light from the analysis substrate 30.

The reference position detection sensor 6 detects a notch 31 of the analysis substrate 30, and generates a reference position detection signal KS to outputs the signal to the signal processing circuit 10. The reference position detection signal KS is, for example, a pulse signal that rises and is in an ON state when the notch 31 reaches the position detected by the reference position detection sensor 6, that is, a position irradiated with the detection light 6a. Alternatively, the reference position detection signal KS falls and is in an OFF state when the notch 31 passes the position.

In other words, the reference position detection sensor 6 detects a reference position for each rotation cycle and track of the analysis substrate 30. A transmission type optical sensor may be used as the reference position detection sensor 6. In this case, the reference position detection sensor 6 irradiates, with the detection light 6a, the analysis substrate 30. Then, the reference position detection sensor 6 receives the detection light 6a passing the notch 31 to detect the reference position for each rotation cycle and track of the analysis substrate 30.

The guide shaft 7 is disposed parallel to the analysis substrate 30, and along a radial direction of the analysis substrate 30. The optical pickup 8 is supported by the guide shaft 7. The optical pickup 8 has an objective lens 81. The optical pickup drive circuit 9 controls driving of the optical pickup 8. The optical pickup drive circuit 9 moves the optical pickup 8 along the guide shaft 7, and moves the objective lens 81 of the optical pickup 8 in the vertical direction. The optical pickup 8 is driven along the guide shaft 7, in a direction orthogonal to the rotation axis C2 of the turntable 2, in the radial direction of the analysis substrate 30, and in parallel with the analysis substrate 30.

The optical pickup 8 irradiates the analysis substrate 30 with a laser beam 82. The laser beam 82 is condensed by the objective lens 81 to a track region 32 where the reaction region 40 on the analysis substrate 30 is formed. While the analysis substrate 30 is rotated, the optical pickup 8 is driven in the radial direction of the analysis substrate 30. The optical pickup 8 receives the reflected light from the analysis substrate 30. The optical pickup 8 detects a light reception level of the reflected light to generate a light reception level signal JS, and outputs the generated signal to the signal processing circuit 10.

The control unit 20 controls the turntable drive circuit 5, the optical pickup drive circuit 9, and the signal processing circuit 10. The control unit 20 controls the turntable drive circuit 5 to rotate the turntable 2, for example, at a constant linear velocity, or stop the turntable 2. The control unit 20 controls the optical pickup drive circuit 9 to move the optical pickup 8 to a target position in the radial direction of the analysis substrate 30. The control unit 20 further controls the circuit to adjust a vertical position of the objective lens 81, so that the laser beam 82 is condensed in the track region 32. The control unit 20 outputs a measurement parameter SP to the signal processing circuit 10. A computing device or a CPU (Central Processing Unit) may be used as the control unit 20.

The signal processing circuit 10 is, for example, a counter, and has a partial region count value computation unit 11 (a first computation unit). The control unit 20 has a relative count value computation unit 21 (a second computation unit). Computation processes of the partial region count value computation unit 11 and the relative count value computation unit 21 will be described later.

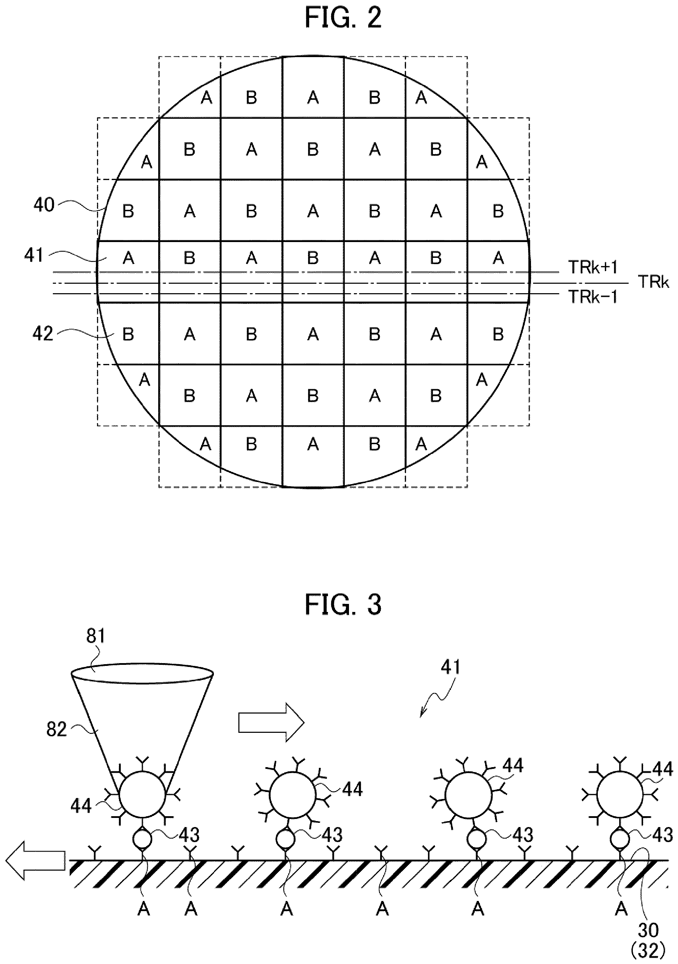

As shown in FIG. 2, the reaction region 40 on the analysis substrate 30 includes a plurality of detection target substance capturing regions 41 (first regions) and a plurality of blank regions 42 (second regions or detection target substance non-capturing regions). The detection target substance capturing regions 41 are fixed with an antibody A (a first antibody) that reacts with a specific antigen of a detection target substance. The blank regions 42 are fixed with an antibody B that does not react with the specific antigen of the detection target substance. The second region is a region in the reaction region 40 other than the first region.

The plurality of detection target substance capturing regions 41 and the plurality of blank regions 42 are formed to be uniformly dispersed in the reaction region 40 so as not to overlap one another. FIG. 2 shows a state in which each of the detection target substance capturing regions 41 and each of the blank regions 42 are alternately formed in a track direction (a left-right direction in FIG. 2) and in a direction orthogonal to the track direction (an up-down direction in FIG. 2).

For example, by incubating the analysis substrate 30 while a solution containing the antibody A is applied to a region corresponding to the detection target substance capturing region 41 on the analysis substrate 30, the plurality of detection target substance capturing regions 41 on the analysis substrate 30 can be formed. By incubating the analysis substrate 30 while a solution containing the antibody B is applied to a region corresponding to the blank region 42 on the analysis substrate 30, the plurality of blank regions 42 on an analysis substrate 30 can be formed.

As shown in FIG. 3, in the detection target substance capturing region 41, the antibody A is fixed on the analysis substrate 30, a detection target substance 43 (a first detection target substance) is captured by the antibody A, and then, fine particles 44 are captured, the fine particles 44 being fixed with an antibody reacting with the specific antigen of the detection target substance 43. The fine particles 44 are count target substances for labeling the detection target substance 43. That is, in the detection target substance capturing region 41, the detection target substance 43 is captured on the analysis substrate 30 by the antibody A and the fine particles 44 in a sandwiched manner. The first detection target substance (the detection target substance 43) is an exosome in which an antigen reacting with the antibody A is expressed on a surface.

The blank region 42 is fixed with the antibody B which does not react with the detection target substance 43. Therefore, although the detection target substance 43 is not bound on the analysis substrate 30, partial fine particles 44 may be bound on the analysis substrate 30.

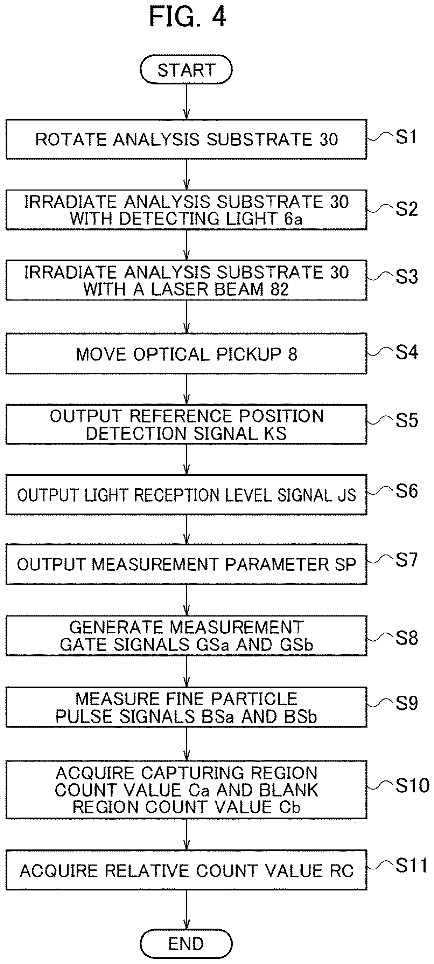

With reference to a flowchart shown in FIG. 4, and FIGS. 5 to 7, an example of procedures until a relative count value is calculated of an analysis method of one embodiment will be described. In step S1 of FIG. 4, the control unit 20 controls the turntable drive circuit 5 such that the analysis substrate 30 rotates at a constant linear velocity, for example, and causes the turntable drive unit 4 to rotate and drive the turntable 2.

In step S2, the control unit 20 causes the reference position detection sensor 6 to irradiate, with the detection light 6, the analysis substrate 30. In step S3, the control unit 20 causes the optical pickup 8 to irradiate, with the laser beam 82, the analysis substrate 30. The control unit 20 may perform step S3 after step S2, step S2 after step S3, or may perform step S2 and step S3 at the same time.

A case will be described in which a kth track TRk shown in FIG. 2 is scanned with the laser beam 82. In step S4, the control unit 20 controls the optical pickup drive circuit 9 to move the optical pickup 8 such that the track TRk of the analysis substrate 30 is irradiated with the laser beam 82. In step S5, the reference position detection sensor 6 generates the reference position detection signal KS by detecting the notch 31, and outputs the generated signal to the signal processing circuit 10.

In step S6, the optical pickup 8 receives the reflected light from the analysis substrate 30. The optical pickup 8 detects a light reception level of the reflected light to generate a light reception level signal JS, and outputs the generated signal to the signal processing circuit 10. In step S7, the control unit 20 outputs the measurement parameter SP to the signal processing circuit 10. The measurement parameter SP is a measurement parameter for generating a plurality of measurement gate signals GS. The measurement parameter SP includes measurement information such as the number of reaction regions 40, a time corresponding to a distance from the notch 31 to each of the reaction regions 40, and a timing and a gate width of the measurement gate signals GS in each track.

The signal processing circuit 10 acquires the reference position detection signal KS from the reference position detection sensor 6, acquires the light reception level signal JS from the optical pickup 8, and acquires the measurement parameter SP and track information from the control unit 20. The track information TF includes information of a target track (a track to be measured) which is irradiated with the laser beam 82 in step S4 above (for example, information such as a track number, a track position or the like). The signal processing circuit 10 acquires the information on the track TRk from the control unit 20.

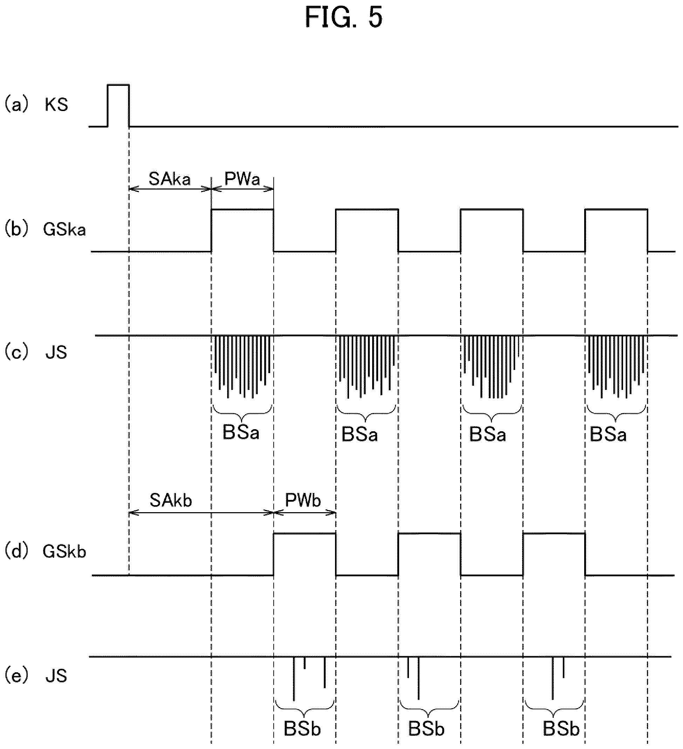

In (a) to (e) of FIG. 5, a vertical axis represents a signal level, and a horizontal axis represents a time axis. The (a) of FIG. 5 shows the reference position detection signal KS. In step S8, the signal processing circuit 10 acquires the phase SA in the track to be measured TR based on the reference position detection signal KS and the track information. The phase SA is preset for each track. The signal processing circuit 10 may include a storage unit that stores the phase SA set for each track as table format data. The signal processing circuit 10 can recognize, from the track information, a track number or a track position of a currently measured track, and acquire the phase SA corresponding to the track.

Further, the signal processing circuit 10 generates the measurement gate signal GS based on the reference position detection signal KS and the phase SA. For example, the signal processing circuit 10 acquires the phase SAk in the track TRk, and generates the measurement gate signal GSk based on the reference position detection signal KS and the phase SAk.

As shown in (b) of FIG. 5, the signal processing circuit 10 acquires the phase SAka in the track TRk, and generates a measurement gate signal GSka (a first gate signal) for measuring the detection target substance capturing regions 41 in the track TRk based on the reference position detection signal KS and the phase SAka. The measurement gate signal GSka is a gate pulse signal which rises when the laser beam 82 reaches the detection target substance capturing regions 41 and falls when the laser beam passes the detection target substance capturing regions 41 in the track TRk. That is, a pulse width PWa of the measurement gate signal GSka corresponds to a time period during which the laser beam 82 is scanned on the detection target substance capturing regions 41.

As shown in (d) of FIG. 5, the signal processing circuit 10 acquires a phase SAkb in the track TRk. The signal processing circuit 10 generates a measurement gate signal GSkb (a second gate signal) for measuring a blank region 42 in the track TRk based on the reference position detection signal KS and the phase SAkb. The measurement gate signal GSkb is a gate pulse signal that rises when the laser beam 82 reaches the blank region 42 and falls when the laser beam 82 passes the blank region 42 in the track TRk. That is, a pulse width PWb of the measurement gate signal GSkb corresponds to a time period during which the laser beam 82 is scanned on the blank region 42.

As shown in (c) of FIG. 5, in step S9, the signal processing circuit 10 extracts a fine particle pulse signal BSa (a first detection signal) from the light reception level signal JS output from the optical pickup 8 during a time period from a rising time to a falling time of the measurement gate signal GSka (corresponding to the pulse width PWa). Then, the signal processing circuit 10 counts the pulse number in the fine particle pulse signal BSa. The fine particle pulse signals BSa are a pulse group extracted from the light reception level signal JS during a time period from a rising time to a falling time of the measurement gate signal GSka. The detection target substance 43 (the fine particles 44) captured in the detection target substance capturing regions 41 is counted based on the pulse number in the fine particle pulse signal BSa. That is, the signal processing circuit 10 counts the detection target substance 43 by counting the fine particles 44 that label the detection target substance 43 (the exosome).

As shown in (e) of FIG. 5, the signal processing circuit 10 further extracts a fine particle pulse signal BSb (a second detection signal) from the light reception level signal JS output from the optical pickup 8 during a time period from a rising time to a falling time of the measurement gate signal GSkb (corresponding to the pulse width PWb). Then, the signal processing circuit 10 counts the pulse number in the fine particle pulse signal BSb. The fine particle pulse signals BSb are a pulse group extracted from the light reception level signal JS during a time period from a rising time to a falling time of the measurement gate signal GSkb.

The blank region 42 is fixed with the antibody B which does not react with the detection target substance 43. Therefore, in the blank region 42, the detection target substance 43 is not bound on the analysis substrate 30. However, the partial fine particles 44 may be bound on the analysis substrate 30.

As shown in (e) of FIG. 5, if the partial fine particles 44 are bound on the analysis substrate 30 in the blank region 42, the signal processing circuit 10 detects the partial fine particles 44 as the fine particle pulse signal BSb from the light reception level signal JS.

The control unit 20, the reference position detection sensor 6, the optical pickup 8, or the signal processing circuit 10 performs the processes of steps S4 to S9 for all the tracks TR in the reaction region 40, totals a count value for each detection target substance capturing region 41, and totals a count value for each blank region 42.

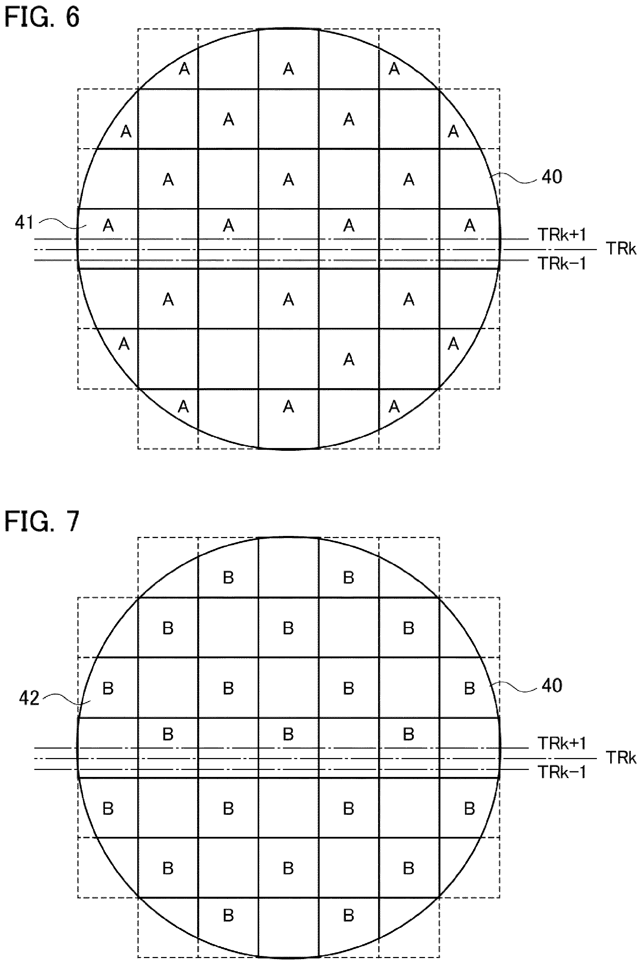

As shown in FIG. 6, in step S10, the partial region count value computation unit 11 totals count values in all of the detection target substance capturing regions 41 of the reaction region 40, and calculates the sum as capturing region count values Ca (first count values). That is, the capturing region count values Ca are count values of the fine particles 44 captured in the detection target substance capturing regions 41 of the reaction region 40. Hereinafter, the capturing region count value Ca is simply referred to as a count value Ca. The partial region count value computation unit 11 calculates an average value per unit area from the count values in all of the detection target substance capturing regions 41 of the reaction region 40, and may set the average value as the count value Ca. The partial region count value computation unit 11 may set the value as an average value per one partial region, if areas of divided individual partial regions are all the same.

As shown in FIG. 7, the partial region count value computation unit 11 adds the count values in all of the blank regions 42 of the reaction region 40, and calculates the sum as a blank region count value Cb (a second count value). Hereinafter, the blank region count value Cb is simply referred to as a count value Cb. The partial region count value computation unit 11 calculates an average value per unit area from the count values in all of the blank regions 42 of the reaction region 40 to set the average value as the count value Cb. The partial region count value computation unit 11 may use the value as an average value per blank region, if areas of the individual divided blank regions are all the same. Further, the partial region count value computation unit 11 outputs the count values Ca and Cb to the relative count value computation unit 21.

In step S11, the relative count value computation unit 21 divides the count value Ca by the count value Cb to calculate a relative count value RC in the reaction region 40. The relative count value computation unit 21 may output the relative count value RC to a display device or the like. The analysis device 1 may include a display device, or alternatively may use an external display device. The analysis device 1 performs processes from steps S1 to S11 above for all of the reaction regions 40 formed on the analysis substrate 30, and then ends the processes.

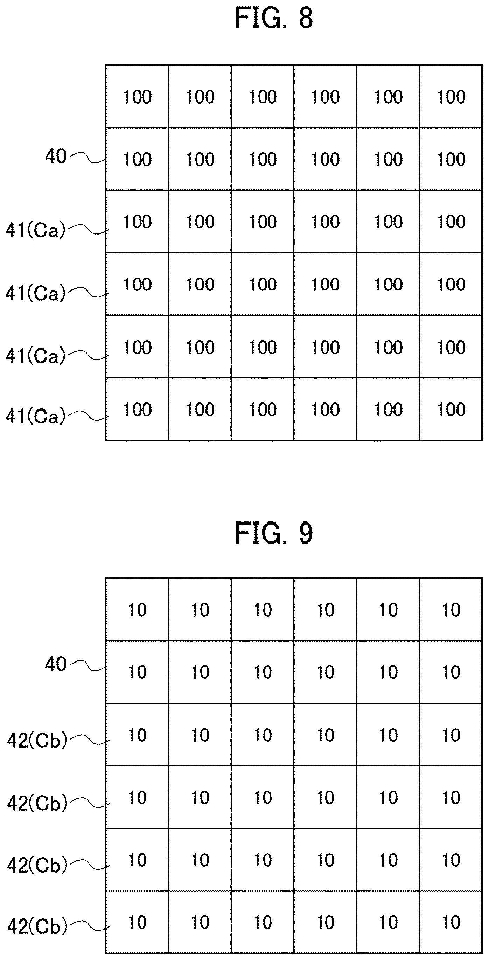

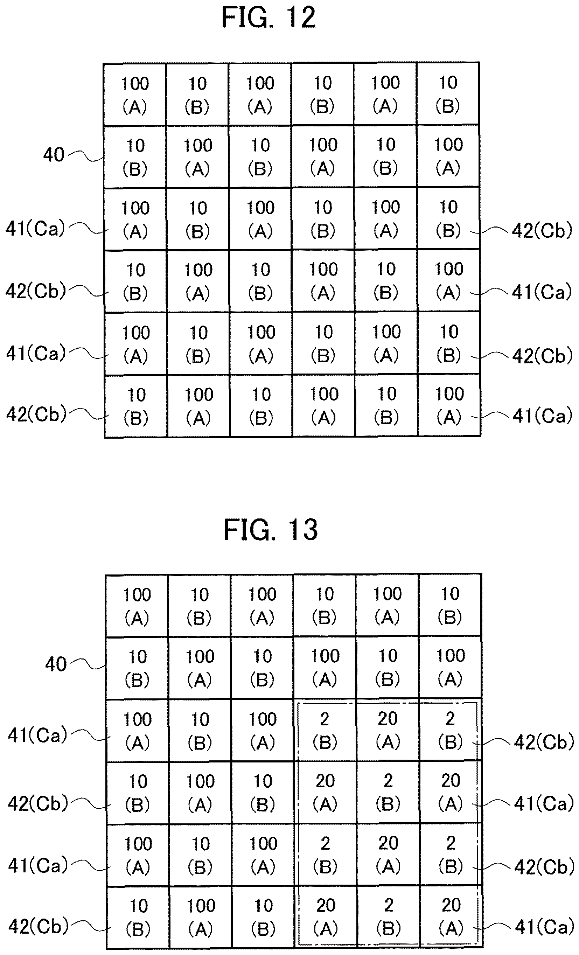

With reference to FIGS. 8 to 13, function effects of the analysis device and the analysis method of the present embodiment will be described. FIGS. 8 to 11 show comparative examples, and FIGS. 12 and 13 show examples. In FIGS. 8 to 13, for clarity of descriptions, the reaction region 40 is set to have a square shape, and is divided into 36 (6 × 6) regions. The count values Ca or Cb in each of the divided regions is shown.

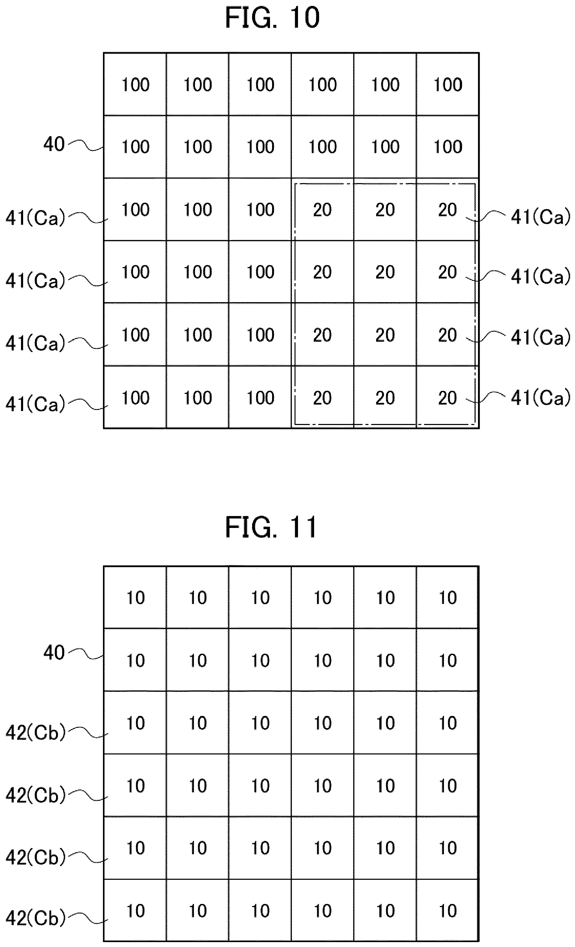

FIGS. 8 and 10 show the count value Ca in each of the divided region if the reaction region 40 is formed only of the detection target substance capturing regions 41. FIGS. 9 and 11 show the count value Cb in each of the divided regions if the reaction region 40 is formed only of the blank regions 42. That is, in the comparative examples, the relative count value RC is calculated by using the two reaction regions 40.

As shown in FIGS. 8 and 9, if the in-plane distributions of the count values Ca and Cb in the detection target substance capturing regions 41 and the blank regions 42 are respectively uniform, the relative count value RC is 10 (Ca (100 × 36)/Cb (10 × 36)). FIG. 10 corresponds to FIG. 8, and FIG. 11 corresponds to FIG. 9. As shown in FIGS. 10 and 11, suppose that the percentage of the count values Ca in lower right regions indicated by a dash-dot-dash line of the detection target substance capturing regions 41 is 20% of the count values Ca of other region. Further, suppose that the in-plane distribution of the count values Cb in the blank regions 42 is uniform. In the above case, the relative count value RC is about 7.3 (Ca (100 × 24 + 20 × 12)/Cb (10 × 36)). That is, in the comparative examples, the relative count value RC differs depending on the in-plane distribution of the count values in the reaction region 40.

FIG. 12 corresponds to FIGS. 8 and 9, and shows that the reaction region 40 includes the detection target substance capturing regions 41 and the blank regions 42. FIG. 12 also shows that the in-plane distributions of the count values Ca and the count values Cb in the detection target substance capturing regions 41 and the blank regions 42 are respectively uniform. The relative count value RC is 10 (Ca (100 × 18)/Cb (10 × 18)).

FIG. 13 corresponds to FIGS. 10 and 11, and shows that the reaction region 40 includes the detection target substance capturing regions 41 and the blank regions 42. Further, FIG. 13 shows that the percentages of the count values Ca and Cb of lower right regions indicated by a dash-dot-dash line of the reaction region 40 are 20% of the count values Ca and Cb of other region respectively. The relative count value RC is 10 (Ca (100 × 12 + 20 × 6)/Cb (10 × 12 + 2 × 6)). That is, the example reduces the influence of the in-plane distribution of the count values in the reaction region 40, and enables the calculation of a uniform relative count value RC or a relative count value RC with less variation than that in the comparative examples.

In the analysis device and the analysis method of the present embodiment, the analysis substrate 30 on which the reaction region 40 is formed is used. The reaction region 40 includes the detection target substance capturing regions 41 and the blank regions 42. The detection target substance capturing regions 41 are fixed with the antibody A reacting with the specific antigen of the detection target substance 43. The blank regions 42 are regions fixed with the antibody B that does not react with the specific antigen of the detection target substance 43, or are regions in which the antibodies A and B are not fixed. The blank regions are also referred to as detection target substance non-capturing regions.

According to the analysis device and the analysis method of the present embodiment, the count value Ca in the detection target substance capturing region 41 and the count value Cb in the blank region 42 are acquired, and then, the count value Ca is divided by the count value Cb. This reduces the influence of the in-plane distribution of the count values in the reaction region 40, and enables the calculation of a uniform relative count value RC, or a relative count value RC with a small variation.

Therefore, according to the analysis device and the analysis method of the present embodiment, even if how an antibody is fixed is different in each of the reaction regions, the analysis can be performed with high accuracy. Although the descriptions were made above that the blank regions 42 are fixed with the antibody B which does not react with the specific antigen of the detection target substance 43, antibodies may not be fixed to the blank regions 42.

The present invention is not limited to the above-described embodiment, and various modifications can be made without departing from the scope of the present invention.

FIG. 2 shows the reaction region 40 in which each of the detection target substance capturing regions 41 and each of the blank regions 42 are alternately formed in a track direction and a direction orthogonal to the track direction. The arrangements of the detection target substance capturing regions 41 and the blank regions 42 are not limited to those shown in FIG. 2, and may be set appropriately.

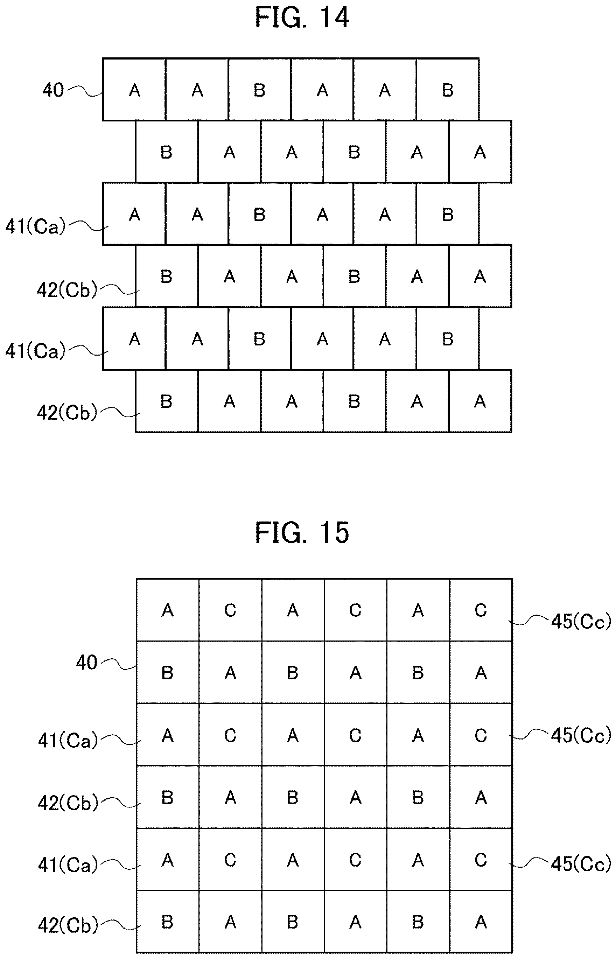

As shown in FIG. 14, in the reaction region 40, the arrangement may be made such that the number of the detection target substance capturing regions 41 is larger than the number of the blank regions 42. Further the detection target substance capturing regions 41 and the blank regions 42 may be arranged by shifting the regions for each row or each column.

As shown in FIG. 15, the reaction region 40 on the analysis substrate 30 may have a plurality of detection target substance capturing regions 41 and 45 formed with different antibodies, and a plurality of blank regions 42. An antibody C (a second antibody) is fixed in the plurality of detection target substance capturing regions 45 (third regions), the antibody C reacting with a specific antigen of a detection target substance (a second detection target substance) that is different from the detection target substance 43. The second detection target substance is an exosome in which an antigen reacting with the antibody C is expressed on a surface. The third region is a region in which the antibody C is fixed in the second region. A region other than the third region in the second region is defined as a fourth region. That is, the plurality of detection target substance capturing regions and the plurality of blank regions may be uniformly dispersed in the reaction region 40 such that the regions do not overlap one another. The number of detection target substance capturing regions, the number of blank regions, and a type of an antibody for each of the detection target substance capturing regions can be appropriately set.

In step S10, the partial region count value computation unit 11 totals the count values in all of the detection target substance capturing regions 41 of the reaction region 40 to calculate the count value Ca. Further, the partial region count value computation unit 11 totals the count values in all of the detection target substance capturing regions 45 of the reaction region 40 to calculate a count value Cc (a third count value). Still further, the partial region count value computation unit 11 totals the count values in all of the blank regions 42 of the reaction region 40 to calculate the count value Cb. The partial region count value computation unit 11 outputs the individual count value Ca to Cc to the relative count value computation unit 21.

The blank regions 42 are fixed with the antibody B which does not react with specific antigens of the first and second detection target substances.

The first count value (the capturing region count value Ca) is a count value of the first detection target substance (the detection target substance 43) that is captured on the reaction region 40 by the antibody A. The first count value is also a count value of the first region (the detection target substance capturing regions 41). The second count value (the blank region count value Cb) is a count value of a region fixed with the antibody B that is the second region (the blank region 42).

The third count value (the count value Cc) is a count value of the second detection target substance captured on the reaction region 40 by the antibody C. The third count value is also a count value of the third region (the detection target substance capturing region 45). The count value of the fourth region is defined as a fourth count value.

The relative count value computation unit 21 divides the count value Ca by the count value Cb in step S11 to calculate the relative count value RC of the detection target substance 43 that is captured by the antibody A and the fine particles 44 in the reaction region 40 in a sandwiched manner. Further, the relative count value computation unit 21 divides the count value Cc by the count value Cb to calculate the relative count value RC of a detection target substance different from the detection target substance 43 that is captured by the antibody C and the fine particles 44 in the reaction region 40 in a sandwiched manner. Therefore, according to the analysis device and the analysis method of the present embodiment, a plurality of detection target substances can be accurately calculated from a single reaction region 40.

The relative count value computation unit 21 may divide the count value Ca by the count value Cc to calculate the relative count value RC of the detection target substance 43 that is captured by the antibody A and the fine particles 44 in the reaction region 40 in a sandwiched manner without using the count value Cb in the blank regions 42. For example, if the antibody A specifically reacts only with the exosome associated with a certain disease, and if the antibody C specifically reacts with the exosome as a whole, the proportion of the disease-specific exosomes in the total exosome can be assessed.

Of course, the reaction region 40 has only the first region and the third region, and the relative evaluation may be performed by using only two types of antibodies. In this case, the detection target substance capturing regions 41 fixed with the antibody A is the first region, and the detection target substance capturing region 45 fixed with the antibody C is the second region. That is, the relative count value computation unit 21 may calculate the relative count value RC in the reaction region 40 based on at least two of the first count value, the third count value, and the fourth count value.

In the prior art, there has been a case where a region with a large count value obtained by counting the fine particles in a single reaction region, and a region with a small count value are mixed together. This affects the result of the quantitative determination, and prevents performing of the accurate analysis. The region with the small count value is caused by the removal of the detection target substance (for example, the exosome) or the fine particles captured in the reaction region from the analysis substrate, for example, by the influence of the injection or aspiration of a solution used in the process of forming the reaction region, or washing, and the like. The region with the large count value was caused by, for example, the counting of noise components such as the residual dross of a solution or a cleaning liquid. According to the analysis device and the analysis method of the present embodiment, even if how an antibody is fixed is different in each reaction region, the analysis can be performed with high accuracy.

The descriptions have been made that in the present embodiment, the signal processing circuit 10 has the partial region count value computation unit 11, and the control unit 20 has the relative count value computation unit 21. However, the signal processing circuit 10 may have the partial region count value computation unit 11 and the relative count value computation unit 21. Alternatively, the control unit 20 may have the partial region count value computation unit 11 and the relative count value computation unit 21.

The present disclosure relates to the subject matter described in Japanese Patent Application No. 2019-056241 filed on March 25, 2019.

## Claims

1. An analysis device (1) comprising:
an analysis substrate (30) on which a plurality of reaction regions (40) are formed, the reaction regions (40) including a first region (41) and a second region (42) that are arranged in a checkered pattern so that the first region (41) and the second region (42) do not overlap each other, the first region (41) being fixed with a first antibody (A) that is specifically bound with a first detection target substance (43) to be captured in the first region (41), and the second region (42) being a region other than the first region (41) in the reaction region (40) and being fixed with a second antibody (B) that is not specifically bound with the first detection target substance (43) to be captured in the first region (41);
an optical pickup (8) configured to irradiate the analysis substrate (30) with a laser beam (82) and receives reflected light from the analysis substrate (30) to generate a light reception level signal (JS), the analysis substrate (30) being bound with the first detection target substance (43) which is reacted with the first region (41) and the second region (42) and being bound with a count target substance which is bound with the first detection target substance (43) captured by the reaction;
a signal processing circuit (10) configured to extract a pulse signal indicating the count target substance from the light reception level signal (JS), and counts a pulse number in the pulse signal; and
a control unit (20) configured to control the optical pickup (8) so as to scan the analysis substrate (30); wherein
the signal processing circuit (10) is configured to generate a first gate signal corresponding to a time period for scanning the first region (41), and a second gate signal corresponding to a time period for scanning the second region (42), and comprises a first computation unit (11) configured to calculate a first count value (Ca) in the first region (41) based on the first gate signal and the pulse signal, and configured to calculate a second count value (Cb) in the second region (42) based on the second gate signal and the pulse signal;
the control unit (20) comprises a second computation unit configured to calculate a relative count value (Rc) in the reaction region (40) based on the first count value (Ca) and the second count value (Cb), and
the first count value (Ca) is calculated by totaling count values in a plurality of the first regions (41), and the second count value (Cb) is calculated by totaling count values in a plurality of the second regions (42).

2. The analysis device (1) according to claim 1, wherein
the second computation unit is configured to calculate the relative count value (Rc) by dividing the first count value (Ca) by the second count value (Cb).

3. The analysis device (1) according to any one of claims 1 to 3, wherein
the count target substance is further bound with a second detection target substance;
the second region (42) includes a third region fixed with a second antibody that is bound with the second detection target substance, and a fourth region other than the third region in the second region (42);
the analysis substrate (30) is bound with the first detection target substance (43) and the second detection target substance which are reacted with the first region (41), the third region, and the fourth region, and is bound with a count target substance which is bound with the first detection target substance (43) and the second detection target substance captured by the reaction;
the first computation unit (11) is configured to calculate the first count value (Ca), a third count value in the third region, and a fourth count value in the fourth region; and
the second computation unit is configured to calculate the relative count value (Rc) based on at least two of the first count value (Ca), the third count value, and the fourth count value.

4. An analysis method comprising:
irradiating an analysis substrate (30) with a laser beam (82) and receiving reflected light from the analysis substrate (30) to generate a light reception level signal (JS), the analysis substrate (30) being bound with a first detection target substance (43) which is reacted with a reaction region (40) and being bound with a count target substance which is bound with the first detection target substance (43) captured by the reaction, the reaction region (40) including a first region (41) fixed with a first antibody (A) which is specifically bound with the first detection target substance (43) to be captured in the first region (41) and a second region (42) which is a region other than the first region (41) in the reaction region (40), the first region (41) and the second region (42) being arranged in a checkered pattern so that the first region (41) and the second region (42) do not overlap each other in the reaction region (40) and is fixed with a second antibody (B) that is not specifically bound with the first detection target substance (43) to be captured in the first region (41);
extracting a pulse signal indicating the count target substance from the light reception level signal (JS), and counting a pulse number in the pulse signal;
calculating a first count value (Ca) obtained by counting the pulse number in the first region (41), and a second count value (Cb) obtained by counting the pulse number in the second region (42); and
calculating, by a second computation unit, a relative count value (Rc) in the reaction region (40) based on the first count value (Ca) and the second count value (Cb), wherein
the first count value (Ca) is calculated by totaling count values in a plurality of the first regions (41), and the second count value (Cb) is calculated by totaling count values in a plurality of the second regions (42).

## Patentansprüche

1. Analysevorrichtung (1), umfassend:
ein Analysesubstrat (30), auf dem eine Vielzahl von Reaktionsbereichen (40) gebildet sind, wobei die Reaktionsbereiche (40) einen ersten Bereich (41) und einen zweiten Bereich (42) umfassen, die in einem Schachbrettmuster angeordnet sind, so dass der erste Bereich (41) und der zweite Bereich (42) einander nicht überlappen, wobei der erste Bereich (41) mit einem ersten Antikörper (A) fixiert ist, der spezifisch an eine erste Detektionszielsubstanz (43) gebunden ist, die im ersten Bereich (41) eingefangen werden soll, und der zweite Bereich (42) ein anderer Bereich als der erste Bereich (41) im Reaktionsbereich (40) ist und mit einem zweiten Antikörper (B) fixiert ist, der nicht spezifisch an die ersten Detektionszielsubstanz (43) gebunden ist, die im ersten Bereich (41) eingefangen werden soll;
einen optischen Aufnehmer (8), der zum Bestrahlen des Analysesubstrats (30) mit einem Laserstrahl (82) konfiguriert ist und reflektiertes Licht vom Analysesubstrat (30) empfängt, um ein Lichtempfangspegelsignal (JS) zu erzeugen, wobei das Analysesubstrat (30) an die erste Detektionszielsubstanz (43) gebunden ist, die mit dem ersten Bereich (41) und dem zweiten Bereich (42) zur Reaktion gebracht wird, und an eine Zählzielsubstanz gebunden ist, die an die erste Detektionszielsubstanz (43) gebunden ist, die durch die Reaktion eingefangen wird;
eine Signalverarbeitungsschaltung (10), die zum Extrahieren eines Pulssignals konfiguriert ist, das die Zählzielsubstanz aus dem Lichtempfangspegelsignal (JS) anzeigt, und eine Pulsanzahl im Pulssignal zählt; und
eine Steuereinheit (20), die zum Steuern des optischen Aufnehmers (8) konfiguriert ist, um das Analysesubstrat (30) zu scannen; wobei
die Signalverarbeitungsschaltung (10) zum Erzeugen eines ersten Gate-Signals, das einem Zeitraum zum Scannen des ersten Bereichs (41) entspricht, und eines zweiten Gate-Signals, das einem Zeitraum zum Scannen des zweiten Bereichs (42) entspricht, konfiguriert ist und eine erste Berechnungseinheit (11) umfasst, die zum Berechnen eines ersten Zählwerts (Ca) im ersten Bereich (41) basierend auf dem ersten Gate-Signal und dem Pulssignal konfiguriert ist und zum Berechnen eines zweiten Zählwerts (Cb) im zweiten Bereich (42) basierend auf dem zweiten Gate-Signal und dem Pulssignal konfiguriert ist;
die Steuereinheit (20) eine zweite Berechnungseinheit umfasst, die zum Berechnen eines relativen Zählwerts (Rc) im Reaktionsbereich (40) basierend auf dem ersten Zählwert (Ca) und dem zweiten Zählwert (Cb) konfiguriert ist, und
der erste Zählwert (Ca) durch Summieren von Zählwerten in einer Vielzahl der ersten Bereiche (41) berechnet wird und der zweite Zählwert (Cb) durch Summieren von Zählwerten in einer Vielzahl der zweiten Bereiche (42) berechnet wird.

2. Analysevorrichtung (1) nach Anspruch 1, wobei
die zweite Berechnungseinheit zum Berechnen des relativen Zählwerts (Rc) durch Dividieren des ersten Zählwerts (Ca) durch den zweiten Zählwert (Cb) konfiguriert ist.

3. Analysevorrichtung (1) nach einem der Ansprüche 1 bis 3, wobei
die Zählzielsubstanz ferner an eine zweite Detektionszielsubstanz gebunden ist;
der zweite Bereich (42) einen dritten Bereich, der mit einem zweiten Antikörper fixiert ist, der an die zweite Detektionszielsubstanz gebunden ist, und einen anderen vierten Bereich als den dritten Bereich im zweiten Bereich (42) umfasst;
das Analysesubstrat (30) an die erste Detektionszielsubstanz (43) und die zweite Detektionszielsubstanz gebunden ist, die mit dem ersten Bereich (41), dem dritten Bereich und dem vierten Bereich zur Reaktion gebracht werden, und an eine Zählzielsubstanz gebunden ist, die an die erste Detektionszielsubstanz (43) und die zweite Detektionszielsubstanz gebunden ist, die durch die Reaktion eingefangen wird;
die erste Berechnungseinheit (11) zum Berechnen des ersten Zählwerts (Ca), eines dritten Zählwerts im dritten Bereich und eines vierten Zählwerts im vierten Bereich konfiguriert ist; und
die zweite Berechnungseinheit zum Berechnen des relativen Zählwerts (Rc) basierend auf zumindest zwei von dem ersten Zählwert (Ca), dem dritten Zählwert und dem vierten Zählwert konfiguriert ist.

4. Analyseverfahren, umfassend:
Bestrahlen eines Analysesubstrats (30) mit einem Laserstrahl (82) und Empfangen von reflektiertem Licht vom Analysesubstrat (30), um ein Lichtempfangspegelsignal (JS) zu erzeugen, wobei das Analysesubstrat (30) an eine erste Detektionszielsubstanz (43) gebunden ist, die mit einem Reaktionsbereich (40) zur Reaktion gebracht wird, und an eine Zählzielsubstanz gebunden ist, die an die erste Detektionszielsubstanz (43) gebunden ist, die durch die Reaktion eingefangen wird, wobei der Reaktionsbereich (40) einen ersten Bereich (41), der mit einem ersten Antikörper (A) fixiert ist, der spezifisch an die erste Detektionszielsubstanz (43) gebunden ist, die in dem ersten Bereich (41) eingefangen werden soll, und einen zweiten Bereich (42) beinhaltet, der ein anderer Bereich als der erste Bereich (41) im Reaktionsbereich (40) ist, wobei der erste Bereich (41) und der zweite Bereich (42) in einem Schachbrettmuster angeordnet sind, so dass der erste Bereich (41) und der zweite Bereich (42) einander im Reaktionsbereich (40) nicht überlappen und mit einem zweiten Antikörper (B) fixiert ist, der nicht spezifisch an die erste Detektionszielsubstanz (43) gebunden ist, die in dem ersten Bereich (41) eingefangen werden soll;
Extrahieren eines Pulssignals, das die Zählzielsubstanz aus dem Lichtempfangspegelsignal (JS) anzeigt, und Zählen einer Pulsanzahl im Pulssignal;
Berechnen eines ersten Zählwerts (Ca), der durch Zählen der Pulsanzahl im ersten Bereich (41) erhalten wird, und eines zweiten Zählwerts (Cb), der durch Zählen der Pulsanzahl im zweiten Bereich (42) erhalten wird; und
Berechnen, durch eine zweite Berechnungseinheit, eines relativen Zählwerts (Rc) im Reaktionsbereich (40) basierend auf dem ersten Zählwert (Ca) und dem zweiten Zählwert (Cb), wobei
der erste Zählwert (Ca) durch Summieren von Zählwerten in einer Vielzahl der ersten Bereiche (41) berechnet wird und der zweite Zählwert (Cb) durch Summieren von Zählwerten in einer Vielzahl der zweiten Bereiche (42) berechnet wird.

## Revendications

1. Dispositif d'analyse (1) comprenant :
un substrat d'analyse (30) sur lequel une pluralité de régions de réaction (40) sont formées, les régions de réaction (40) comprenant une première région (41) et une seconde région (42) qui sont agencées selon un motif en damier de sorte que la première région (41) et la seconde région (42) ne se chevauchent pas, la première région (41) étant fixée avec un premier anticorps (A) qui est spécifiquement lié à une première substance cible de détection (43) à capturer dans la première région (41), et la seconde région (42) étant une région autre que la première région (41) dans la région de réaction (40) et étant fixée avec un second anticorps (B) qui n'est pas spécifiquement lié à la première substance cible de détection (43) à capturer dans la première région (41) ;
un capteur optique (8) configuré pour irradier le substrat d'analyse (30) avec un faisceau laser (82) et reçoit une lumière réfléchie à partir du substrat d'analyse (30) pour générer un signal de niveau de réception de lumière (JS), le substrat d'analyse (30) étant lié à la première substance cible de détection (43) qui réagit avec la première région (41) et la seconde région (42) et étant lié à une substance cible de comptage qui est liée à la première substance cible de détection (43) capturée par la réaction ;
un circuit de traitement de signal (10) configuré pour extraire un signal d'impulsion indiquant la substance cible de comptage à partir du signal de niveau de réception de lumière (JS), et compte un nombre d'impulsions dans le signal d'impulsion ; et
une unité de commande (20) configurée pour commander le capteur optique (8) de manière à balayer le substrat d'analyse (30) ; dans lequel
le circuit de traitement de signal (10) est configuré pour générer un premier signal de grille correspondant à une période de temps pour balayer la première région (41), et un second signal de grille correspondant à une période de temps pour balayer la seconde région (42), et comprend une première unité de calcul (11) configurée pour calculer une première valeur de comptage (Ca) dans la première région (41) sur la base du premier signal de grille et du signal d'impulsion, et configurée pour calculer une seconde valeur de comptage (Cb) dans la seconde région (42) sur la base du second signal de grille et du signal d'impulsion ;
l'unité de commande (20) comprend une seconde unité de calcul configurée pour calculer une valeur de comptage relative (Rc) dans la région de réaction (40) sur la base de la première valeur de comptage (Ca) et de la seconde valeur de comptage (Cb), et
la première valeur de comptage (Ca) est calculée en totalisant des valeurs de comptage dans une pluralité des premières régions (41), et la seconde valeur de comptage (Cb) est calculée en totalisant des valeurs de comptage dans une pluralité des secondes régions (42).

2. Dispositif d'analyse (1) selon la revendication 1, dans lequel
la seconde unité de calcul est configurée pour calculer la valeur de comptage relative (Rc) en divisant la première valeur de comptage (Ca) par la seconde valeur de comptage (Cb).

3. Dispositif d'analyse (1) selon l'une quelconque des revendications 1 à 3, dans lequel
la substance cible de comptage est en outre liée à une seconde substance cible de détection ;
la seconde région (42) comprend une troisième région fixée avec un second anticorps qui est lié à la seconde substance cible de détection, et une quatrième région autre que la troisième région dans la seconde région (42) ;
le substrat d'analyse (30) est lié à la première substance cible de détection (43) et à la seconde substance cible de détection qui réagissent avec la première région (41), la troisième région et la quatrième région, et est lié à une substance cible de comptage qui est liée à la première substance cible de détection (43) et à la seconde substance cible de détection capturée par la réaction ;
la première unité de calcul (11) est configurée pour calculer la première valeur de comptage (Ca), une troisième valeur de comptage dans la troisième région et une quatrième valeur de comptage dans la quatrième région ; et
la seconde unité de calcul est configurée pour calculer la valeur de comptage relative (Rc) sur la base d'au moins deux de la première valeur de comptage (Ca), de la troisième valeur de comptage et de la quatrième valeur de comptage.

4. Procédé d'analyse comprenant :
irradier un substrat d'analyse (30) avec un faisceau laser (82) et recevoir une lumière réfléchie à partir du substrat d'analyse (30) pour générer un signal de niveau de réception de lumière (JS), le substrat d'analyse (30) étant lié à une première substance cible de détection (43) qui réagit avec une région de réaction (40) et étant lié à une substance cible de comptage qui est liée à la première substance cible de détection (43) capturée par la réaction, la région de réaction (40) comprenant une première région (41) fixée avec un premier anticorps (A) qui est spécifiquement lié à la première substance cible de détection (43) à capturer dans la première région (41) et une seconde région (42) qui est une région autre que la première région (41) dans la région de réaction (40), la première région (41) et la seconde région (42) étant agencées dans un motif en damier de sorte que la première région (41) et la seconde région (42) ne se chevauchent pas dans la région de réaction (40) et est fixée avec un second anticorps (B) qui n'est pas spécifiquement lié à la première substance cible de détection (43) à capturer dans la première région (41) ;
extraire un signal d'impulsion indiquant la substance cible de comptage à partir du signal de niveau de réception de lumière (JS), et compter un nombre d'impulsions dans le signal d'impulsion ;
calculer une première valeur de comptage (Ca) obtenue en comptant le nombre d'impulsions dans la première région (41), et une seconde valeur de comptage (Cb) obtenue en comptant le nombre d'impulsions dans la seconde région (42) ; et
calculer, par une seconde unité de calcul, une valeur de comptage relative (Rc) dans la région de réaction (40) sur la base de la première valeur de comptage (Ca) et de la seconde valeur de comptage (Cb), dans lequel
la première valeur de comptage (Ca) est calculée en totalisant des valeurs de comptage dans une pluralité des premières régions (41), et la seconde valeur de comptage (Cb) est calculée en totalisant des valeurs de comptage dans une pluralité des secondes régions (42).
